# EUROPEAN PATENT APPLICATION

(11) **EP 4 741 419 A1**
(43) Date of publication of application: **13.05.2026**
(21) Application number: 24838456.2
(22) Date of filing: 21.05.2024
(51) Int. Cl.: C07K 14/47, C12N 15/12, C12N 15/63, A61K 38/17, A61P 11/00, A61P 29/00, A61P 25/08, A61P 31/12, A61P 9/12

(54) **KERATIN CF4, PREPARATION METHOD THEREFOR, PHARMACEUTICAL COMPOSITION COMPRISING SAME, AND USE THEREOF**

(30) Priority: 07.07.2023 CN 202310829280
(71) Applicant: Institute of Materia Medica, Chinese Academy of Medical Sciences, Beijing 100050 (CN)
(72) Inventor: YU, Shishan, Beijing 100050 (CN); WANG, Xiaoliang, Beijing 100050 (CN); MA, Shuanggang, Beijing 100050 (CN); WANG, Xiaojing, Beijing 100050 (CN); QU, Jing, Beijing 100050 (CN); WANG, Ling, Beijing 100050 (CN); FENG, Nan, Beijing 100050 (CN); LI, Mi, Beijing 100050 (CN); XU, Shaofeng, Beijing 100050 (CN); CAI, Jie, Beijing 100050 (CN); LI, Yong, Beijing 100050 (CN); WANG, Weiping, Beijing 100050 (CN); LIU, Yunbao, Beijing 100050 (CN); ZHANG, Mi, Beijing 100050 (CN)
(74) Representative: EDP Patent Attorneys B.V.
(86) International application number: PCT/CN2024/094396
(87) International publication number: WO 2025/011197

(57) **Abstract**

Provided are keratin CF4, a preparation method therefor, a pharmaceutical composition containing same, and a use thereof, relating to the technical field of medicines. Specifically, provided are keratin CF4, a nucleic acid molecule encoding the keratin CF4, an expression vector containing the nucleic acid molecule, a host cell containing the expression vector or a genome into which the nucleic acid molecule is integrated, a preparation method for the keratin CF4, a pharmaceutical composition containing the keratin CF4, and a use of the keratin CF4, the nucleic acid molecule, the expression vector, the host cell or the pharmaceutical composition in preparation of medicaments for antipyretic, analgesic, antitussive, expectorant, anticonvulsant, antiepileptic, antihypertensive, anti-inflammatory, or antiviral purpose.

## Description

### TECHNICAL FIELD

The present invention relates to the technical field of medicines, and relates to a keratin CF4, a nucleic acid molecule encoding keratin CF4, an expression vector containing the nucleic acid molecule, a host cell containing the expression vector or with the nucleic acid molecule genomically integrated, preparation methods for keratin CF4 and pharmaceutical compositions containing such keratin, and uses of said keratin and the pharmaceutical composition in the preparation of medicaments for antipyretic and analgesic, antitussive and expectorant, anticonvulsant, antiepileptic, antihypertensive, anti-inflammatory, and antiviral purposes.

### BACKGROUND OF THE INVENTION

Keratin is a type of protein, which is widely found in the epidermis of humans and animals, and is the main component of hair, feathers, hoofs, shells, claws, horns, etc. It is a highly important structural protein in connective tissues and plays a role in protecting the body.

Keratin is widely present in organisms and is a renewable resource with great utilization value, but it has not been widely and effectively utilized. The main reason is that keratin is insoluble in various solvents, and keratin is generally more resistant to protease digestion than other proteins. Therefore, it is very difficult to extract and prepare natural keratin.

With the rapid development of modern biotechnology such as genomics, proteomics, genetic engineering, and microbial engineering, an increasing number of genes have been identified, and the use of protein expression systems to prepare and produce target proteins is an important method for studying the biological functions of genes or proteins.

No literature has reported the use of a protein expression system to prepare the target keratin and subsequently study its structure and function, demonstrating novelty and inventiveness.

### SUMMARY OF THE INVENTION

The technical problem solved by the present invention is to provide a keratin CF4, a nucleic acid molecule encoding the keratin CF4, an expression vector containing the nucleic acid molecule, and a host cell containing the expression vector or genome integrating the nucleic acid molecule, and a preparation method of the keratin CF4, a pharmaceutical composition containing the keratin CF4, and use of the above-mentioned keratin CF4, nucleic acid molecule, expression vector, host cell, or pharmaceutical compositions in the preparation medicament for antipyretic, analgesic, antitussive, expectorant, anticonvulsant, antiepileptic, antihypertensive, anti-inflammatory or antiviral.

To solve the technical problems of the present invention, the present invention provides the following technical solutions:
The first aspect of the technical solution of the present invention is to provide a keratin CF4, characterized in that the amino acid sequence of the keratin CF4 is:
(1) the amino acid sequence shown in SEQ ID NO: 1 in the sequence listing.
(2) an amino acid sequence that substantially maintains the same biological function as the keratin CF4 formed by substitution, deletion or addition of 1-35 amino acids to the amino acid sequence set forth in SEQ ID NO:1 in the sequence listing.

Further, the keratin CF4 may have a conventional modification; or the keratin CF4 is further linked with a tag for detection or purification.

Furthermore, the conventional modification includes acetylation, amidation, cyclization, glycosylation, phosphorylation, alkylation, biotinylation, fluorescent group modification, polyethylene glycol PEG modification, immobilization modification, sulfation, oxidation, methylation, deamination, formation of disulfide bond or breakage of disulfide bond; the tag includes His6, GST, EGFP, MBP, Nus, HA, IgG, FLAG, c-Myc, and Profinity eXact.

The second aspect of the technical solution of the present invention provides a nucleic acid molecule encoding the keratin CF4 of the first aspect.

Further, the nucleotide sequence of the nucleic acid molecule is:
(1) the nucleotide sequence shown in SEQ ID NO:2 in the sequence listing.
(2) a nucleotide sequence obtained by sequence optimization based on the nucleotide sequence shown in SEQ ID NO:2.
(3) a nucleotide sequence complementary to the nucleotide sequence of (1) or (2) above.

The third aspect of the technical solution of the present invention provides an expression vector, which is characterized in that the expression vector contains the nucleic acid molecule described in the second aspect.

Further, the expression vector may be pET series, pUC series, pQE series, pBV series, pMAL series, pPIC9, pPIC9K, pHIL-S1, pPICZα/A, pYAM75P, pHIL-D2, pA0815, pPIC3K, pPICZ, pHWO10, pGAPZ, pGAPZa, pPIC3.5K, etc.; the preferred expression vector is the pET series vector; the most preferred expression vector is pET-28a(+).

The fourth aspect of the technical solution of the present invention provides a host cell, characterized in that the host cell contains the expression vector of the third aspect or the nucleic acid molecule of the second aspect integrated into the genome.

Further, the host cell is selected from the group consisting of bacterium, yeast, aspergillus, plant cell, and insect cell.

Furthermore, the bacterium is *Escherichia coli* or yeast.

Competent host cell can be BL21 series, Transetta series, Rosetta series, DH5α series, JM series, Top series, Origami series, Trans1-T1, TG1, TB1; Y11430, MG1003, GS115 (AOX1), KM71, SMD1168, etc.; The preferred expression competent cell is BL21 (DE3), or Transetta (DE3).

The fifth aspect of the technical solution of the present invention provides a method for preparing the keratin CF4 of the first aspect, which is characterized in that it comprises the following steps:
A. synthesizing a nucleic acid molecule corresponding to the keratin CF4 described in the first aspect, ligating the nucleic acid molecule into an appropriate expression vector, and transforming the expression vector into a host cell. Culturing the host cell harboring the expression vector under appropriate conditions in a fermentation device and inducing the expression of the keratin CF4 to obtain a crude protein solution containing the keratin CF4.
B. subjecting the crude protein solution obtained in step A to separation and purification, and drying to obtain the keratin CF4.

Further, in step A, the host cell is mainly selected from *Escherichia coli,* the keratin CF4 is expressed in the inclusion bodies of *Escherichia coli,* and the fermentation device includes shaker flask or fermenter.

Further, in step A, after inducing the expression of the keratin CF4, impurities may be washed away with washing solution, followed by dissolution with a urea solution to obtain a crude protein solution.Further, the medium in step A may be LB medium, TB medium, SB medium, SOB medium, SOC medium, PDA medium, YPD medium, red Bengal medium, high salt Chashi medium, DOBA medium, rice koji medium, and modified formula thereof, etc.; shake flask fermentation preferably LB medium, TB medium, most preferably TB medium; fermenter preferably LB medium and modified formula thereof.

Further, the inducer in step A may be IPTG, lactose, arabinose, etc.; Preferred is IPTG or lactose.

Further, in step A, the obtained fermentation broth is centrifuged and then the supernatant is discarded; the precipitate is suspended in a buffer, the bacteria are broken, centrifuged again, and the supernatant is discarded; after the precipitate is washed with a washing solution, which is then dissolved in a urea solution to obtain the crude protein solution of CF4.

Among them, the buffer is preferably buffer A, and its dosage is: fermentation broth volume: buffer A volume = 1~100:1, preferably 10:1;
The washing solution may be urea solution, guanidine hydrochloride solution, Triton and buffer A, etc., preferably urea solution, most preferably 2M urea solution (which may contain 1% Triton), and 4M urea solution. The dosage is: fermentation broth volume: urea solution volume = 0.2~100:1, preferably 1~15:1;
The urea solution is preferably 4M~8M urea solution, most preferably 8M urea solution and its dosage is: fermentation broth volume:8M urea volume = 0.2~100: 1, preferably 2~15: 1.

Further, in step B, the method of separation and purification includes ultrafiltration microfiltration membrane technology purification method, column chromatography purification method, salting out method, and dialysis method.

Further, in step B, the method of separation and purification is as follows:
(1) The dialysis method is to purify the crude protein solution obtained in step A by a dialysis method to obtain the target protein CF4 solution.
   The molecular weight cut-off of the dialysis bag may be 0.5-10 kD, the preferred molecular weight cut-off of the dialysis bag is 3.5-10 kD, and the most preferred molecular weight cut-off of the dialysis bag is 10 kD.
(2) In the ultrafiltration and microfiltration method, the crude protein solution obtained in step A is purified by membrane technology such as ultrafiltration membrane or microfiltration membrane to obtain a concentrated solution of the target protein CF4.
(3) The column chromatography method is to pass the crude protein solution obtained in step A through column chromatography, such as various exchange columns or exclusion column chromatography, to separate and purify the target protein CF4.

The preferred exclusion column is dextran gel column, SUPERDEX 30 INCREASE, SUPERDEX 75 INCREASE, SUPERDEX 200 INCREASE, and SUPEROSE 6 INCREASE, etc.. The preferred exchange column is an ion exchange resin column: anion exchange resin column: HITRAP Q FF, HITRAP CAPTO Q ImpRes, CAPTO Q ImpRes, HITRAP CAPTO Q, HITRAP DEAE, TOYOPEARL Q-650M and TOYOPEARL SUPER Q-650M, etc.; Cation exchange resin column: HITRAP SP FF, HITRAP CAPTO SP ImpRes, Capto SP ImpRes, HITRAP CAPTO SP, TOYOPEARL SP-650M and TOYOPEARL SUPER SP-650M. The most preferred is an anion exchange resin column.

As the eluent, commonly used eluents in the art may be used, such as water and salt solution. The salt solution includes sodium chloride solution, sodium dihydrogen phosphate solution, disodium hydrogen phosphate solution, sodium acetate, acetic acid, and the like.

(4) The salting-out method is to purify the crude protein solution obtained in step A by salting-out method to obtain the target protein CF4 suspension.

The salting-out agent may be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of salting out is 1 to 3 times, preferably 2 times.

After salting out, the precipitate is washed with pure water, and the washing frequency is 2 to 5 times, preferably 3 times.

Further, the target protein CF4 solution purified in step B may be freeze-dried or vacuum-dried into a dry powder, or the concentrated solution may be directly spray-dried into a dry powder.

The sixth aspect of the technical solution of the present invention provides a pharmaceutical composition, characterized in that the pharmaceutical composition contains the keratin CF4 described in the first aspect or the nucleic acid molecule described in the second aspect or the expression vector of the third aspect or the host cell of the fourth aspect and a pharmaceutically acceptable carrier or excipient.

The keratin obtained in the above steps of the present invention may be freeze-dried or vacuum-dried into a dry powder, or the concentrated liquid may be directly spray-dried into a dry powder, and then made into various dosage forms.

The present invention relates to a pharmaceutical composition, which comprises any keratin obtained in the above steps and a pharmaceutically acceptable carrier.

The present invention also relates to a pharmaceutical composition containing the keratin of the present invention as an active ingredient and conventional pharmaceutical excipients or adjuvants. Generally, the keratin of the present invention accounts for 0.1-100.0% of the total weight of the pharmaceutical composition.

The present invention also provides a pharmaceutical composition, which includes a pharmaceutical effective dose of protein as an active ingredient and a pharmaceutically acceptable carrier.

The pharmaceutical composition of the present invention may be prepared according to methods recognized in the field. When used for this purpose, if necessary, the protein of the present invention may be combined with one or more solid or liquid pharmaceutical excipients and/or adjuvants to prepare an appropriate administration form or dosage that may be used as human or veterinary drugs form.

The keratin of the present invention or the pharmaceutical composition containing the same may be administered in a unit dosage form. The route of administration may be enteral or parenteral, such as oral administration, intramuscular, subcutaneous, nasal cavity, oral mucosa, eyes, lungs, skin, vagina, peritoneum, and rectum, etc., oral administration is preferred.

The keratin protein of the present invention or the pharmaceutical composition containing the same may be administered by injection. Injection includes intravenous injection, intramuscular injection, subcutaneous injection, intradermal injection, intraperitoneal injection, and acupoint injection, etc..

The dosage form for administration may be a liquid dosage form, a solid dosage form, or a semi-solid dosage form. Liquid dosage form may be solution (including true solutions and colloidal solutions), emulsion (including oil-in-water, water-in-oil, and double emulsion), suspension, injection (including water injection, powder injection, and infusion), eye drops Lotion, nasal drops, lotion, and liniment, etc. The solid dosage form may be tablet (including ordinary tablet, enteric-coated tablet, buccal tablet, dispersible tablet, chewable tablet, effervescent tablet, and orally disintegrating tablet), capsule (including hard capsule, soft capsule, and enteric-coated capsule), granules preparation, powder, pellet, dripping pill, suppositorie, film, patche, air (powder) spray, spray, etc.; semi-solid dosage form may be ointment, gel, paste, etc.

The keratin of the present invention may be made into ordinary preparations, slow-release preparations, controlled-release preparations, targeted preparations, and various particle delivery systems.

To make a unit dosage form into a tablet, various excipients known in the art may be widely used, including diluents, binders, humectants, disintegrants, lubricants, and glidants. The diluent may be starch, dextrin, sucrose, glucose, lactose, mannitol, sorbitol, xylitol, microcrystalline cellulose, calcium sulfate, calcium hydrogen phosphate, calcium carbonate, etc.; the humectant may be water, ethanol, iso propanol, etc.; the binder may be starch syrup, dextrin, syrup, honey, glucose solution, microcrystalline cellulose, acacia syrup, gelatin syrup, sodium carboxymethyl cellulose, methyl cellulose, hypromellose Base cellulose, ethyl cellulose, acrylic resin, carbomer, polyvinylpyrrolidone, polyethylene glycol, etc.; the disintegrant may be dry starch, microcrystalline cellulose, low-substituted hydroxypropyl cellulose, cross-linked polyvinylpyrrolidone, cross-linked sodium carboxymethyl cellulose, sodium carboxymethyl starch, sodium bicarbonate and rafter acid, calcium carbonate, polyoxyethylene sorbitol fatty acid ester, dodecyl sodium sulfonate; lubricant and glidant may be talc, silicon dioxide, stearate, tartaric acid, liquid paraffin, polyethylene glycol, etc.

The tablets can also be further made into coated tablets, such as sugar-coated tablets, film-coated tablets, enteric-coated tablets, or double-layer tablets and multi-layer tablets.

To make the administration unit into a pill, various carriers known in the field may be widely used. Examples of carriers are, for example, diluents and absorbents, such as glucose, lactose, starch, cocoa butter, hydrogenated vegetable oil, polyvinylpyrrolidone, polyethylene glycol laurate, kaolin, talc, etc.; binders, such as Gum arabic, xanthan gum, gelatin, ethanol, honey, liquid sugar, rice paste or batter, etc.; disintegrants, such as agar powder, dried starch, alginate, sodium lauryl sulfonate, methylcellulose, ethyl cellulose and so on.

To make the administration unit into a suppository, various carriers known in the field may be widely used. Examples of carriers are, for example, polyethylene glycol, lecithin, cocoa butter, higher alcohols, higher alcohol esters, gelatin, semi-synthetic glycerides, and the like.

To make the dosing unit into a capsule, the active ingredient keratin of the present invention is mixed with the above-mentioned various carriers, and then the resulting mixture is filled into hard gelatin capsules or soft capsules. Alternatively, the active ingredient keratin may be formulated as microcapsules, suspended in an aqueous medium to form a suspension, or filled into hard capsules or prepared as injections for administration.

For example, the keratin of the present invention is prepared into injection preparations, such as solutions, suspension solutions, emulsions, and freeze-dried powder injections. Such preparations may be aqueous or non-aqueous and may contain one and/or more pharmacodynamically acceptable carriers, diluents, binders, lubricants, preservatives, surfactants, or dispersants. For example, the diluent may be selected from water, ethanol, polyethylene glycol, 1,3-propylene glycol, ethoxylated isostearyl alcohol, polyoxylated isostearyl alcohol, polyoxyethylene sorbitol fatty acid esters and the like. In addition, in order to prepare an isotonic injection, an appropriate amount of sodium chloride, glucose or glycerin may be added to the injection preparation. In addition, conventional solubilizers, buffers, pH adjusters, etc. can also be added. These auxiliary materials are commonly used in this field.

In addition, if necessary, coloring agents, preservatives, flavors, flavors, sweeteners or other materials can also be added to the pharmaceutical preparations.

In order to achieve the purpose of medication and enhance the therapeutic effect, the keratin or the pharmaceutical composition of the present invention may be administered by any known administration method.

The dosage of the keratin pharmaceutical composition of the present invention depends on many factors, such as the nature and severity of the disease to be prevented or treated, the gender, age, weight, personality and individual response of the patient or animal, the route of administration, the number of administrations and the purpose of treatment, so the therapeutic dose of the present invention can have a wide range of changes. Generally speaking, the dosage of the pharmaceutical ingredients of the present invention is well known to those skilled in the art. Appropriate adjustments may be made according to the actual amount of the drug contained in the final preparation of the keratin composition of the present invention to meet the requirement of the therapeutically effective amount and accomplish the preventive or therapeutic purpose of the present invention. The appropriate daily dosage range of keratin of the present invention: the dosage of keratin of the present invention is 0.01~500 mg/kg body weight, preferably 0.5~100 mg/kg body weight, more preferably 1~50 mg/kg body weight, and most preferably 2~30 mg/kg body weight. The above dosage may be administered in a single dosage form or divided into several, such as two, three, or four dosage forms, depending on the clinical experience of the administering doctor and the dosage regimens including the use of other treatments. The total dose required for each treatment may be divided into multiple or single doses. The protein or pharmaceutical composition of the present invention may be taken alone, or used in combination with other therapeutic drugs or symptomatic drugs and adjust the dose.

The seventh aspect of the technical solution of the present invention provides the use of the keratin CF4 according to the first aspect or the nucleic acid molecule according to the second aspect or the expression vector according to the third aspect or the host cell according to the fourth aspect or the pharmaceutical composition according to the sixth aspect in the preparation of medicament for antipyretic, analgesic, antitussive, expectorant, anticonvulsant, antiepileptic, antihypertensive, anti-inflammatory, or antiviral.

To accomplish the purpose of the present invention, the present invention provides the following technical solutions. Specifically, the preparation of the keratin CF4 of the present invention includes the following steps:
(1) Synthesizing the nucleotide sequence and determining the accuracy of the sequence;
   The preferred nucleotide sequence is shown in SEQ ID NO:2.
(2) Transferring the nucleotide sequence into an expression vector;
   The expression vector may be pET series, pUC series, pQE series, pBV series, pMAL series, pPIC9K, pHIL-S1, pPICZα/A, pYAM75P, pHIL-D2, pA0815, pPIC3K, pPICZ, pHWO10, pGAPZ, pGAPZa, pPIC3.5K etc. The preferred expression vector is the pET series vector; the most preferred expression vector is pET-28a(+).
(3) Transfection of expression vector into host cell;
   The host cell may be *E. coli* or yeast; the preferred host cell is *E. coli*;
   Competent cell may be BL21 series, Transetta series, Rosetta series, DH5α series, JM series, Top series, Origami series, Trans1-T1, TG1, TB1; Y11430, MG1003, GS115 (AOX1), KM71, SMD1168, etc. Preferred expression competent cell is BL21(DE3) or transetta (DE3).
(4) Culturing the host cell under appropriate conditions to induce the expression of the target protein CF4;
   Fermentation device can use shake flask or fermenter;
   The medium may be LB medium, TB medium, SB medium, SOB medium, SOC medium, PDA medium, YPD medium, red Bengal medium, high salt Chashi medium, DOBA medium, rice koji culture medium, and their improved formulas, etc.; Shake flask fermentation is preferably LB medium, TB medium, and most preferably TB medium; fermenter preferably LB medium and its improved formulas.
   The inducer may be IPTG, lactose, arabinose, etc.; preferably is IPTG or lactose.
(5) Enriching the target protein CF4 product;
   Centrifuge the fermentation broth obtained in step (4), and discard the supernatant; suspend the precipitate in the buffer, then crush the bacteria, and then centrifuge again, and discard the supernatant; after washing the precipitate with a washing solution, dissolved with urea solution to obtain CF4 crude protein solution.
   Among them, the buffer is preferably buffer A, and its dosage is: fermentation broth volume: buffer A volume = 1~100: 1, preferably 5~10: 1;
   The washing solution may be urea solution, guanidine hydrochloride solution, Triton, buffer A, etc., preferably urea solution, most preferably 2M urea solution (which may contain 1% Triton). The dosage is fermentation broth volume: urea solution volume = 0.2~100: 1, preferably 1~15: 1. The washing procedure is performed 3~10 times, preferably 6 times.
   The urea solution is preferably a 4M~8M urea solution, most preferably 8M urea solution. Its dosage is fermentation broth volume: 8M urea volume = 0.2~100:1, preferably 2~15: 1.
(6) Separation and purification of target protein CF4.

The crude protein solution obtained in step (5) needs to be purified to obtain the target protein CF4. The purification may be carried out by dialysis, ultrafiltration/ microfiltration, column chromatography, or salting out steps.
A. In the dialysis step, the crude protein solution obtained in step (5) is purified by a dialysis method to obtain the target protein CF4 solution.
   The molecular weight cut-off of the dialysis bag may be 0.5-10 kD, the preferred molecular weight cut-off of the dialysis bag is 3.5-10 kD, and the most preferred molecular weight cut-off of the dialysis bag is 10 kD.
B. In the ultrafiltration and microfiltration step, the crude protein solution obtained in step (5) is purified by membrane technology such as ultrafiltration membrane or microfiltration membrane to obtain the concentrated solution of the target protein CF4.
   Preferably, the microfiltration membrane purification is performed twice, with the membrane pore size is 1000~1500nm in the first time, and the membrane pore size is 20~50nm in the second time.
C. In the column chromatography step, the crude protein solution obtained in step (5) is passed through column chromatography, such as various exchange columns or exclusion column chromatography, to separate and purify the target protein CF4.

The preferred exclusion column is the dextran gel column, SUPERDEX 30 INCREASE, SUPERDEX 75 INCREASE, SUPERDEX 200 INCREASE, SUPEROSE 6 INCREASE, etc.; The preferred exchange column is an ion exchange resin column: anion exchange resin column, HITRAP Q FF, HITRAP CAPTO Q ImpRes, CAPTO Q ImpRes, HITRAP CAPTO Q, HITRAP DEAE, TOYOPEARL Q-650M, TOYOPEARL SUPER Q-650M, etc.; Cation exchange resin column, HITRAP SP FF, HITRAP CAPTO SP ImpRes, CAPTO SP ImpRes, HITRAP CAPTO SP, TOYOPEARL SP-650M, TOYOPEARL SUPER SP-650M. The most preferred is an anion exchange resin column.

As the eluent, commonly used eluents in the art may be used, such as water, salt solution, and the salt solution includes sodium chloride solution, sodium dihydrogen phosphate solution, disodium hydrogen phosphate solution, sodium acetate, acetic acid, and the like.

D. The salting-out step is to purify the crude protein solution obtained in step (5) by a salting-out method to obtain the target protein CF4 suspension.

The salting-out agent may be ammonium sulfate, sodium sulfate, sodium chloride, magnesium chloride, aluminum sulfate, ammonium nitrate, ammonium chloride, magnesium sulfate, and the like. The preferred salting-out agent is ammonium sulfate and its aqueous solution. A saturated aqueous solution of ammonium sulfate is added to make the final concentration of ammonium sulfate reach 10-50%, preferably 20-30%, more preferably 25%.

The number of salting out is 1 to 3 times, preferably 2 times.

After salting out, the precipitate is washed with pure water, and the washing frequency is 2 to 5 times, preferably 3 times.

The target protein CF4 solution purified from steps A to D may be freeze-dried or vacuum-dried into dry powder, or the concentrated solution may be directly spray-dried into dry powder.

The beneficial technical effects of the present invention:
1. The protein of the present invention is the keratin obtained for the first time, and the preparation method of the present invention has the characteristics of high yield and high purity.
2. The present invention, through a pharmacodynamic study of protein CF4 on a yeast-induced fever model in SD rats, demonstrates that protein CF4 significantly reduces the elevated body temperature of the model animals at 2 hours post-modeling.
3. The present invention, through a pharmacodynamic study of protein CF4 on pilocarpine (PLO)-induced convulsions and epilepsy in mice, demonstrates that protein CF4 significantly prolongs the latency to Grade II epileptic seizures.
4. The present invention, through a pharmacodynamic study of protein CF4 using the phenol red excretion method in mice, demonstrates that protein CF4 exhibits a significant expectorant effect, which is comparable to that of the positive control drug Mucosolvan (ambroxol).
5. The present invention, through a pharmacodynamic study of protein CF4 on acetic acid-induced writhing in ICR mice, demonstrates that protein CF4 reduces the number of writhes and possesses an analgesic effect.

### DRAWINGS

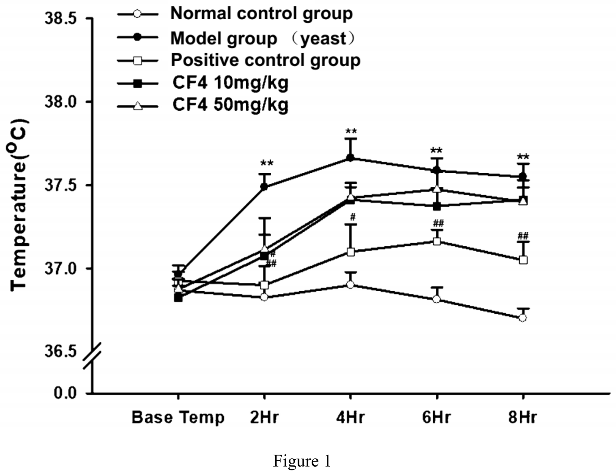

Figure 1: Effect of protein CF4 on yeast-induced fever model in rats.
(Compared with normal control group, ** P<0.01; Compared with the model group, # P<0.05, ## P<0.01).

### DETAILED EMBODIMENTS

The following examples and pharmacological activity test examples are used to further illustrate the present invention, but this does not mean any limitation to the present invention.

The experimental methods in the following examples and pharmacological activity test examples are conventional unless otherwise specified; the experimental materials used, unless otherwise specified, are purchased from conventional biochemical reagent companies.

### Example 1 Preparation of crude protein CF4 solution A by shake flask fermentation (TB medium)

Synthesize the nucleotide sequence shown in SEQ ID NO:2 and transfer it into the pET-28a(+) vector; confirm the sequence to obtain an expression vector containing the correct sequence; transfect the expression vector into BL21(DE3) cells, obtain expression competent host cells containing the target nucleotide sequence. Add LB medium and incubate in a shaker at 37°C and 220 rpm for 1 hour to obtain a recombinant strain.

Dip the recombinant strain into an LBA plate containing Kanamycin, and place the plate upside down in a 37°C constant temperature incubator overnight for 16 hours.

Configure 400 mL of TB medium, divided into 2 bottles, with each bottle of 200 mL. Add Kanamycin (final concentration 50 µg/mL) to each bottle (200 mL) of TB medium. Take a single colony on the plate and add it to the TB medium. Amplify and culture overnight at 37°C and 220 rpm to obtain seed liquid in the shaker.

Configure 28.8 L TB medium, divided into 144 bottles, with each bottle of 200 mL. Add Kanamycin (final concentration 50 µg/mL) to each bottle (200 mL) of TB medium, then add 2 mL of seed solution, and incubate in a shaker at 37°C and 220 rpm for 2-3 hours. Monitor the OD₆₀₀, when the OD₆₀₀ reaches about 1.0, add an inducer to induce protein expression in a shaker, and the induction conditions are selected from the following table.

| | Inducer | Induction temperature | Induction time | Shaker speed |
|---|---|---|---|---|
| Induction conditions | IPTG (Final concentration 0.5 mM) | 16 °C | 16 h | |
| | | 25 °C | 8h | 220 rpm |
| | | 37 °C | 5h | |

Combine each bottle of bacterial liquid, centrifuge at 7000 rpm for 5 minutes, and discard the supernatant after sterilization; The precipitate is suspended in about 3 L of buffer, filtered with an 80-100 mesh screen, and the filtrate is crushed with a high-pressure crusher at a pressure of 800-1000 bar, twice, 2 minutes each time. Centrifuge the broken bacteria liquid at 7000 rpm for 30 minutes, discard the supernatant, and obtain the precipitate (i.e. inclusion body). The precipitate is washed with washing solution (three times with 1 L of 2M urea - 1% Triton solution, followed by two times with 1 L of 3M urea solution), centrifuged, and the supernatant discarded. The precipitate is then dissolved in 1 L of 8M urea solution to obtain the crude protein solution A.

### Example 2 Preparation of crude protein CF4 solution B by shake flask fermentation (other medium)

In Example 1, it was synthesized and sequenced to confirm that an expression vector containing the sequence shown in SEQ ID NO:2 was obtained. The expression vector is transfected into Transetta (DE3) cells to obtain expression-competent host cells containing the target nucleotide sequence.

Prepare 20 mL of LB medium, take 800 µl, add 50 µl of host cells containing the target coding sequence, and incubate at 37 °C and 220 rpm for 1 hour in a shaker.

Dip the above bacterial liquid and streak it in an LBA plate containing Kanamycin, and place the plate upside down in a 37°C constant temperature incubator overnight for 16 hours.

Take 10 mL of LB medium, add Kanamycin (final concentration 50 µg/mL), take a single colony on the plate, and add it to the LB medium. Amplify and culture overnight at 37°C and 220 rpm for 15 hours to obtain seed liquid in a shaker.

Configure 1 L of the medium shown in the table below, and divide it into 10 bottles of 100 mL each. Add Kanamycin (final concentration 50 µg/mL) to each bottle (100 mL) of medium and then add 1 mL of seed solution. Incubate at 37°C and 220 rpm for 2-3 hours in a shaker. Monitor OD₆₀₀ and add inducer IPTG (final concentration 0.5 mM) when OD₆₀₀ reaches about 1.0. Induce protein expression at 37°C and 220 rpm in a shaker.

| | |
|---|---|
| Culture medium | LB medium, SOB medium, SOC medium |

Combine each bottle of bacterial liquid, centrifuge at 10000 rpm for 10 minutes, and discard the supernatant after sterilization; The precipitate is suspended in about 100 mL of buffer, filtered with an 80-100 mesh screen, and the filtrate is crushed with a high-pressure crusher at a pressure of 800-1000 bar, twice, 2 minutes each time. Centrifuge the broken bacteria liquid at 10000 rpm for 30 minutes and discard the supernatant.

The precipitate was first washed with 40 mL of washing solution buffer A, followed by centrifugation and removal of the supernatant. Then, 40 mL of a 2M urea solution (containing 1% Triton) was added to the precipitate for washing. After centrifugation, the supernatant was discarded, and this step was repeated twice. Next, the precipitate was washed twice with 40 mL of a 3M urea solution, each time followed by centrifugation and removal of the supernatant. Finally, 40 mL of an 8M urea solution was added to the precipitate to obtain the crude protein solution B.

### Example 3 Preparation of crude protein CF4 solution C in a fermenter

In Example 1, it was synthesized and sequenced to confirm that an expression vector containing the sequence shown in SEQ ID NO:2 was obtained. The expression vector is transfected into BL21 (DE3) cells to obtain expression-competent host cells containing the target nucleotide sequence. Add LB medium and incubate in a shaker at 37°C and 220 rpm for 1 hour to obtain a recombinant strain.

In the LBA plate containing Kanamycin, add 100 µL of the recombinant strain, spread with the spreader until it becomes evenly dry, and place the plate upside down in a constant temperature incubator at 37°C for overnight culture. Take three single colonies, streak them on a plate containing Kanamycin, and then culture the plate overnight. After three batches of shake flask fermentation and expression verification are confirmed to be correct, the strains are preserved with 15% glycerol and divided into 0.8 mL each to obtain a working cell bank, which is stored in a refrigerator at -80°C for later use.

Take out one tube of glycerol bacteria from the working cell bank, take 100 µl, add it to 40 mL LB medium, add Kanamycin (final concentration 50 µg/mL), and incubate it in a shaker at 37°C and 220 rpm for 6 hours to obtain a first-level seed solution.

Take 1.2 mL of the first-level seed solution, add it to 120 mL LB medium, add Kanamycin (final concentration 50 µg/mL), and then incubate it in a shaker at 37°C and 220 rpm for 7 hours to obtain a second-level seed solution.

Add 3 L of modified LB broth to a 5 L fermentor, followed by 120 mL of second-level seed solution and 3 mL of Kanamycin (final concentration 50 µg/mL), and cultivate at 37°C and 30% dissolved oxygen (series rotation speed) for about 8 hours. When the OD value is around 20, 3g lactose is used as an inducer to induce at 20°C, supplemented at a rate of 30 mL/hour, and incubated at 20°C for 24 hours.

Centrifuge the bacterial solution at 7000 rpm for 5 minutes, sterilize the supernatant, and discard it. The precipitate is suspended in about 600 mL of buffer A, filtered with an 80-100 mesh sieve, and the filtrate is crushed with a high-pressure crusher at a pressure of 800-1000 bar twice for 2 minutes each time. After crushing, centrifuge the bacteria liquid at 7000 rpm for 30 minutes and discard the supernatant.

The precipitate was washed three times with 2M urea solution (containing 1% Triton), using 1 L each time. Then, it was washed with 1 L of 3M urea solution, followed by centrifugation and removal of the supernatant. This step was repeated twice. Next, it was washed once with 1 L of 8M urea solution, centrifuged, and the supernatant was discarded. Finally, 2 L of 8M urea solution was added to the precipitate to obtain the crude protein solution C.

### Example 4 Protein CF4 prepared by dialysis from crude protein solution A

The crude protein solution A obtained in Example 1 was filtered with a 0.45 µm filter membrane, and the filtrate was combined. The filtrate was dialyzed with water, with the molecular weight cut-off of the dialysis bag was 10 kD. After 72 hours of dialysis, the inner liquid was freeze-dried to obtain the target protein CF4.

### Confirmation of the structure of protein CF4 by LC-MS/MS-based complete sequence analysis of the protein

Main materials: acetonitrile, formic acid, ammonium bicarbonate, dithiothreitol (DTT), iodoacetamide (IAA), trypsin, chymotrypsin, Glu-C, Asp-N;
Main instruments: Capillary High Performance Liquid Chromatograph (Thermo Ultimate 3000), Electrospray-Combined Ion Trap Orbitrap Mass Spectrometer (Thermo Q Exative Hybrid Quadrupole-Orbitrap Mass Spectrometer).

### Methods and results:

Protein CF4 undergoes pretreatments such as dissolution replacement, reductive alkylation, and various proteolysis to obtain enzyme-cleaved peptide segments. The solution of the enzyme-cleaved peptide segments was analyzed by liquid chromatography tandem mass spectrometry, and the raw mass spectrometry file uses Maxquant (1.6.2.10) to search the protein database to analyze the data. The identification results confirmed that it was consistent with the target sequence SEQ ID NO:1.

### Example 5 Protein CF4 prepared by purifying crude protein solution A through salting out method.

The crude protein solution A was placed in a stirred container and subjected to two rounds of salting-out. During salting-out, a saturated ammonium sulfate solution was slowly added along the wall of the container until the final concentration reached 25% or 50%, causing protein precipitation. The mixture was then filtered to complete the first salting-out. The protein obtained from the first salting-out was suspended in 400 mL of pure water. A saturated ammonium sulfate solution was again slowly added along the wall until a final concentration of 25% was achieved, performing the second salting-out to obtain a precipitate. After filtration, the crude protein extract was obtained. The crude protein extract was washed three times with water, and then suspended in 200 mL of pure water, stirred, allowed to stand, and filtered. This procedure was repeated three times. The resulting precipitated protein was then freeze-dried to yield the target protein CF4.

The product protein CF4 obtained by the salting out method was confirmed to have the same amino acid sequence as the protein prepared in Example 4 through the same structural confirmation method as in Example 4.

### Example 6 Preparation of protein CF4 by purifying the crude protein solution B

The crude protein solution B obtained in Example 2 was purified by the following two methods:
The first method: dialysis;
The crude protein solution B is filtered through a 0.45 µm membrane, and the filtrate is dialyzed with water for more than 72 hours. The inner solution is then freeze-dried to obtain the target protein CF4.

| | |
|---|---|
| Dialysis bag | Molecular weight cut-off: 0.5kD, 3.5kD, 5kD, 10kD |

The second method: salting out;
The crude protein solution B was placed in a stirred container and subjected to two rounds of salting-out. During salting-out, a saturated ammonium sulfate solution was slowly added along the wall of the container until the final concentration reached 25% or 50%, causing protein precipitation. The mixture was then filtered to complete the first salting-out. The protein obtained from the first salting-out was suspended in 400 mL of pure water. A saturated ammonium sulfate solution was again slowly added along the wall until a final concentration of 25% was achieved, performing the second salting-out to obtain a precipitate. After filtration, the crude protein extract was obtained. The crude protein extract was washed three times with water, and then suspended in 200 mL of pure water, stirred, allowed to stand, and filtered. This procedure was repeated three times. The resulting precipitated protein was then freeze-dried to yield the target protein CF4.

The product protein CF4 obtained by the two methods was confirmed to have the same amino acid sequence as the protein prepared in Example 4 through the same structural confirmation method as in Example 4.

### Example 7 Preparation of protein CF4 by purifying crude protein solution C.

The crude protein solution C is purified by microfiltration membrane technology: use a 20 nm or 50 nm ceramic membrane core for repeated microfiltration to remove urea. The inner liquid is freeze-dried to obtain the target protein CF4.

The product protein CF4 obtained by microfiltration membrane technology was confirmed to have the same amino acid sequence as the protein prepared in Example 4 through the same structural confirmation method as in Example 4.

### Pharmacological test

### Experimental example 1 The pharmacodynamic test of protein CF4 (the protein in Example 7) on the fever model of SD rats induced by yeast

Animals: 230-260 grams of male SD rats;
Medicines: yeast (OXOID LP0021), aspirin (SIGMAA2093), protein CF4;
Instruments: electronic balance (SARTORIUS BP121S), electronic clinical thermometer (CITIZEN CT-513W).

### Experiment grouping:

Normal control group;
Model group: yeast fever model;
Positive control group: Aspirin 300 mg/kg group;
Protein CF4, 10 mg/kg group, 50 mg/kg group.

### Method:

Preparation of experimental animals: After 1 day of acclimatization in the experimental environment (temperature 22°C ± 2°C, relative humidity 50% ± 2%), the experimental animals underwent pre-adaptation to rectal temperature measurement procedures at 8:00 AM and 3:00 PM. Animals were fasted for 12 hours with free access to water prior to experiments. Before temperature measurement, animals were allowed to defecate. The probe of the digital thermometer was lightly coated with petroleum jelly and inserted 2 cm into the rat rectum (marked at 2 cm to ensure consistent depth). Temperature readings were recorded after stabilization.

Subcutaneous injection of dry yeast to establish the rat fever model: Basal body temperature was measured before modeling. Rats with temperatures between 36.2-37.3°C were selected as qualified subjects and randomly divided into groups (n=8 per group). Aspirin or varying doses of protein CF4 were administered orally, immediately followed by subcutaneous injection of 20% yeast suspension (10 mL/kg). The normal control group received an equal volume of saline subcutaneously. Temperature monitoring commenced 2 hours post-modeling and continued at 2-hour intervals for 8 hours.

### Statistics:

According to the body temperature measured at each time point on the day of the experiment, calculate the mean, standard deviation and standard error of the body temperature of each group of rats. The data of each group was compared with t-test, and P<0.05 was considered as a significant difference.

### Experimental results:

Immediately after oral administration of aspirin (300 mg/kg), protein CF4 (10 mg/kg, 50 mg/kg), subcutaneous injection of 20% yeast was performed to establish a model. The animal body temperature was monitored at 2 hours, 4 hours, 6 hours, and 8 hours after modeling. The results are shown in Table 1 and Figure 1.

**Table 1. Effects of the tested drugs on the yeast-induced fever model in rats**

| Group | N | Basal body temperature (°C) | Body temperature 2 hours after modeling (°C) | Body temperature 4 hours after modeling (°C) | Body temperature 6 hours after modeling (°C) | Body temperature 8 hours after modeling (°C) |
|---|---|---|---|---|---|---|
| Normal control group | 8 | 36.9±0.1 | 36.8±0.1 | 36.9±0.1 | 36.8±0.1 | 36.7±0.1 |
| Model group | 8 | 37.0±0.1 | 37.5±0.1** | 37.7±0.1** | 37.6±0.1** | 37.6±0.1** |
| Positive control group | 8 | 36.9±0.1 | 36.9±0.1## | 37.1±0.2# | 37.2±0.1## | 37.1±0.1## |
| CF4 10mg/kg | 8 | 36.8±0.1 | 37.1±0.1# | 37.4±0.1 | 37.4±0.1 | 37.4±0.1 |
| CF4 50mg/kg | 8 | 36.9±0.1 | 37.1±0.2 | 37.4±0.1 | 37.5±0.1 | 37.4±0.1 |

| | | | | | | |
|---|---|---|---|---|---|---|
| (Compared with the normal control group, ** P<0.01; compared with the model group, # P<0.05, ## P<0.01) | | | | | | |

### Experimental results:

Following oral administration of aspirin (300 mg/kg) or protein CF4 (10 mg/kg, 50 mg/kg), a fever model was immediately established by subcutaneous injection of 20% yeast suspension. Body temperature was monitored at 2, 4, 6, and 8 hours post-modeling. The results demonstrated that:
1) The model group exhibited significantly elevated body temperatures at 2, 4, 6, and 8 hours post-modeling compared to the normal group (P < 0.05), indicating statistically significant differences. This confirms the successful establishment of a stable and reliable model.
2) The positive control aspirin group effectively suppressed the yeast-induced temperature increase in model rats at all monitored time points (2, 4, 6, and 8 hours). Significant differences were observed versus the model group (*P <* 0.05), demonstrating consistent efficacy of the positive drug aspirin.
3) The 10 mg/kg dose group of protein CF4 significantly inhibited the rise in body temperature in model rats at 2 hours after modeling. Compared with the model group, the results were statistically significant (P < 0.05).

### Experimental example 2 the pharmacodynamic test of protein CF4 (the protein in Example 7) on convulsive epilepsy in mice caused by the convulsion agent Pilocarpine (PLO)

Animals: male ICR mice;
Drugs: Pilocarpine HCl (PLO, pilocarpine, pilocarpine hydrochloride), Diazepam (diazepam tablets), protein CF4.

### Experiment grouping:

### Model group:

Diazepam (Diazepam) 2mg/kg group;
Protein CF4, 50mg/kg group, 200mg/kg group.

### Method:

### Model preparation and drug administration:

Administration of the test drug began on the afternoon prior to model establishment with a single dose. On the day of modeling, the test drug was administered orally, followed by an intraperitoneal injection of PLO (modeling agent) at 225 mg/kg one hour later. The positive control drug was administered once 20 minutes before modeling. Animals were continuously observed for 30 minutes after PLO injection.
Observation indicators: ① Epileptic seizures: Duration of Grade II-IV seizures; ② Time of death.

Seizure grading criteria (based on Racine scale): Grade 0: No response; Grade I: manifested as twitching of facial muscles or the corners of the mouth; Grade II: Head nodding; Grade III: twitching of one limb; Grade IV: rigidity or body twitching; Grade V: generalized epilepsy (generalized tonic seizures).

### Data processing:

The number of mice exhibiting Grade IV seizures and deaths in each group was statistically analyzed; and the latency periods for Grade II, III, and IV seizures were recorded. For mice that did not progress to Grade IV seizures, the latency was recorded as the maximum value of 1800 seconds. The chi-square test was used for statistical analysis of counts. Mean and standard error were calculated for latency periods. Intergroup comparisons between the model group and other groups were performed using the TTEST. A value of P < 0.05 was considered statistically significant.
Experimental results: see Table 2 and Table 3.

**Table 2. Experiments of tested drugs on PLO-induced epilepsy in mice-statistics of cases**

| Group | Number of experimental examples | Number of Grade IV cases | Grade IV seizure rate | Number of deaths | mortality rate |
|---|---|---|---|---|---|
| Model group | 9 | 7 | 78% | 0 | 0 |
| Diazepam 2 mg/kg | 7 | 0** | 0 | 0 | 0 |
| CF4 50mg/kg | 8 | 8 | 100% | 3 | 38% |
| CF4 200mg/kg | 8 | 7 | 88% | 2 | 25% |

| | | | | | |
|---|---|---|---|---|---|
| (Compared with the model group, ** P<0.01) | | | | | |

**Table 3. Effects of the test drug on the latency of Grade II, III, and IV seizures in epilepsy mice induced by PLO (mean±SEM).**

| Group | Latency to Grade II seizure (s) | Latency to Grade III seizure (s) | Latency to Grade IV seizure (s) | Seizure Grading |
|---|---|---|---|---|
| Model group | 66±4 | 94±4 | 1086±145 | 3.8±0.1 |
| Diazepam 2 mg/kg | 98±7** | 124±8** | 1800±0** | 3.0±0.0** |
| CF4 50 mg/kg | 78±3* | 103±4 | 741±92 | 4.4±0.2 |
| CF4 200 mg/kg | 76±4 | 99±4 | 976±176 | 4.1±0.2 |

| | | | | |
|---|---|---|---|---|
| (Compared with the model group, *P<0.05, **P<0.01) | | | | |

### Experimental results:

1) Experimental results showed that the incidence rate of Grade IV seizures in the model group was 78%, with a mortality rate of 0.
2) The positive control drug can completely suppress the incidence of Grade IV epileptic seizures, and significantly prolong the latency of Grade III and Grade IV epileptic seizures in mice.
3) In the comparison of latency to Grade II epileptic seizures, the CF4 50 mg/kg dose group showed statistically significant differences compared to the model group.

### Experimental example 3 the pharmacodynamic test of protein CF4 (the protein in Example 7) on the expectorant of phenol red excretion method in mice.

Animals: male ICR mice;
Drugs and reagents: Mucosultan (ambroxol hydrochloride tablets), phenol red, sodium bicarbonate, protein CF4;
Instruments: centrifuge (Sigma-3K15), balance (XS105DU), Microplate tester (BIO-TEK).

### Experiment grouping:

Solvent control group;
Mucosultan 30 mg/kg group;
Protein CF4, 20 mg/kg group, 50 mg/kg group.

### Method:

### Model preparation and administration:

Animals were fasted for 16 hours (with free access to water) prior to the experiment. A 30 mg/kg dose of Mucosolvan and different doses of protein CF4 were administered orally (administration volume: 10 mL/kg), respectively. The solvent control group received an equal volume of distilled water. One hour later, a 2.5% phenol red solution was injected intraperitoneally. After 30 minutes, the mice were euthanized by cervical dislocation. Take a trachea from below the thyroid cartilage to the branch of the trachea, and put the trachea into 3 ml 5% NaHCO₃ solution and let it stand for 3 hours. Take 1 ml of the supernatant and centrifuge at 3000 rpm for 5 minutes. Measure and record the absorbance at 546 nm. According to the standard curve of phenol red, the excretion of phenol red was calculated.

### Data processing:

Record the time point of oral administration, the time point of intraperitoneal injection of 2.5% phenol red solution, and the time point of taking the trachea respectively; The absorbance of each group of samples was measured by the microplate reader at 546nm, Calculate the excretion of phenol red according to the standard curve of phenol red. Calculate the mean and standard error of the data in each group, and use t-test to compare the solvent control group with other groups, and P<0.05 is considered as a significant difference.

### Experimental results:

After administration of Mucosolvan (30 mg/kg) and different doses of protein CF4 (20 mg/kg, 50 mg/kg), a 2.5% phenol red solution was injected intraperitoneally one hour later. After 30 minutes, the mice were euthanized by cervical dislocation. Take the trachea from below the thyroid cartilage to the branch of the trachea, put the trachea into 3 ml of 5% NaHCO₃ solution and let it stand for 3 hours, take 1ml of supernatant, centrifuge at 3000rpm for 5 minutes, measure and record the absorbance at 546 nm. According to the standard curve of phenol red, the excretion of phenol red was calculated. The results are shown in Table 4.

**Table 4. Effects of the test drug on the expectorant efficacy in the mouse phenol red excretion model (X±SEM)**

| Group | N | Phenol red excretion (µg/mL) | P |
|---|---|---|---|
| Solvent control group | 9 | 0.537±0.066 | --- |
| Mucosolvan 30 mg/kg | 9 | 0.971±0.082** | 0.001 |
| CF4 20 mg/kg | 9 | 0.830±0.098* | 0.025 |
| CF4 50 mg/kg | 9 | 1.041±0.119** | 0.002 |

| | | | |
|---|---|---|---|
| (Compared with the solvent control group, * P<0.05, ** P<0.01) | | | |

### Experimental results:

1) The experimental results showed that compared with the solvent control group, the amount of phenol red excretion in the Mucosultan 30 mg/kg group was significantly increased, P<0.05, which was statistically significant.
2) Compared to the solvent control group, the 20 mg/kg and 50 mg/kg dose groups of protein CF4 showed a significant increase in phenol red excretion (P < 0.05), indicating a statistically significant difference. The expectorant effect of CF4 was comparable to that of Mucosultan.

### Experimental example 4 Efficacy of protein CF4 (the protein in Example 7) on acetic acid-induced writhing in ICR mice

Animals: male ICR mice;
Drugs and reagents: aspirin, physiological saline, glacial acetic acid, protein CF4.

### Experiment grouping:

Model group;
Aspirin 300 mg/kg group;
Protein CF4, 50 mg/kg group, 200 mg/kg group.

### Method:

One day after the experimental animals adapt to the environment, the experimental animals were orally administered aspirin (300 mg/kg) or protein CF4 (50 mg/kg, 200 mg/kg) one hour in advance (administration volume: 10 ml/kg). Subsequently, a 0.6% acetic acid solution was injected intraperitoneally. The latency (in seconds) and number of writhing episodes exhibited by the animals within 15 minutes were recorded.

### Data Processing:

Calculate the mean and standard error of the data in each group. Compared with the model group by t-test, P<0.05 was considered as statistically different.

### Experimental Results:

One hour after oral administration of aspirin (300 mg/kg) or different doses of protein CF4 (50 mg/kg, 200 mg/kg), a 0.6% acetic acid solution was injected intraperitoneally. The latency and number of writhing episodes in ICR mice were observed. The results are shown in Table 5.

**Table 5. Effects of the test drug on the analgesic efficacy against acetic acid-induced writhing in ICR mice.**

| Group | N | Weight (g) | Writhing latency (seconds) | Number of writhing episodes (times) |
|---|---|---|---|---|
| Model group 0.6% acetic acid | 16 | 24.8±0.1 | 218.4±15.2 | 26.9±3.7 |
| aspirin 300 mg/kg | 14 | 24.9±0.2 | 316.8±34.8* | 11.8±1.8** |
| CF4 50 mg/kg | 12 | 25.3±0.1 | 268.1±59.1 | 25.8±4.8 |
| CF4 200 mg/kg | 12 | 25.1±0.2 | 235.2±23.6 | 15.7±3.0* |

| | | | | |
|---|---|---|---|---|
| (Compared with the model group, * P<0.05, ** P<0.01) | | | | |

### Experimental results:

0.6% acetic acid solution was injected into the abdominal cavity of mice, which caused deep and large area and long-term painful stimulation, causing the mice to writhe (the abdomen was contracted into an "S" shape, the trunk and hind legs were stretched, the buttocks were raised and creeping). The incubation time to start writhing and times of writhing in mice were used as the pain response indexs to determine whether the test sample had analgesic effect. The results of this experiment show:
1) Aspirin 300 mg/kg can significantly delay the incubation period of writhing and reduce the times of writhing, and has a certain analgesic effect. The difference was statistically significant compared to the model group (P < 0.05).
2) The CF4 (200 mg/kg) group showed a trend of delaying the writhing latency and reducing the number of writhing episodes, but the difference was not statistically significant compared to the model group.

## Claims

1. A keratin CF4, wherein the amino acid sequence of the keratin CF4 is:
(1) the amino acid sequence shown as SEQ ID NO:1; or
(2) an amino acid sequence that substantially maintains the same biological function formed by substitution, deletion or addition of 1-45 amino acids to the amino acid sequence set forth in SEQ ID NO:1.

2. The keratin CF4 according to claim 1, wherein the keratin CF4 may have a conventional modification; or the keratin CF4 is further linked with a tag for detection or purification, or the keratin CF4 is a homologous protein thereof.

3. The keratin CF4 according to claim 2, wherein theconventional modification includes acetylation, amidation, cyclization, glycosylation, phosphorylation, alkylation, biotinylation, fluorophore group modification, polyethylene glycol PEG modification, immobilization modification, sulfation, oxidation, methylation, deamidation, disulfide bond formation, or disulfide bond breakage; and the tag includes His6, GST, EGFP, MBP, Nus, HA, IgG, FLAG, c-Myc, and Profinity eXact.

4. A nucleic acid molecule encoding the keratin CF4 according to any one of claims 1-3.

5. The nucleic acid molecule according to claim 4, wherein the nucleotide sequence of the nucleic acid molecule is:
(1) the nucleotide sequence shown as SEQ ID NO:2;
(2) a nucleotide sequence obtained by sequence optimization based on the nucleotide sequence shown as SEQ ID NO: 2; or
(3) a nucleotide sequence complementary to the nucleotide sequence of (1) or (2) above.

6. An expression vector, wherein the expression vector comprises the nucleic acid molecule according to any one of claims 4-5.

7. A host cell, wherein the host cell comprises the expression vector according to claim 6, or has the nucleic acid molecule according to any one of claims 4-5 integrated into its genome.

8. The host cell according to claim 7, wherein the host cell is selected from the group consisting of bacterium, yeast, *Aspergillus,* plant cell, and insect cell.

9. The host cell according to claim 8, wherein the bacterium is *Escherichia coli.*

10. A method for producing the keratin CF4 according to any one of claims 1-3, wherein the method comprises the following steps:
A. synthesizing the nucleic acid molecule corresponding to the keratin CF4 according to any one of claims 1-3, ligating the nucleic acid molecule into a suitable expression vector, transforming the expression vector into a host cell, culturing the host cell harboring the expression vector under specific conditions in a fermentation device, and inducing expression of the keratin CF4 to obtain a crude protein solution containing the keratin CF4;
B. subjecting the crude protein solution obtained in step A to separation and purification, and drying to obtain the keratin CF4.

11. The method according to claim 10, wherein in step A: the host cell is primarily selected from *Escherichia coli*; the keratin CF4 is expressed in the inclusion bodies of *Escherichia coli*; and the fermentation device includes a shake flask or a fermenter.

12. The method according to claim 10, wherein in step A, after inducing the expression of the keratin CF4, a washing solution may be used to wash away impurities, and then the product is dissolved with a solution to obtain the crude protein solution .

13. The method according to claim 10, wherein in step B, the separation and purification method is selected from the group consisting of ultrafiltration microfiltration membrane technology purification method, column chromatography purification method, salting out method, and dialysis method.

14. A pharmaceutical composition, wherein the pharmaceutical composition comprises the keratin CF4 according to any one of claims 1-3 and a pharmaceutically acceptable carrier or excipient.

15. Use of the keratin CF4 according to any one of claims 1-3, or the nucleic acid molecule according to any one of claims 4-5, or the expression vector according to claim 6, or the host cell according to any one of claims 7-9, or the pharmaceutical composition according to claim 14 in the manufacture of a medicament for antipyretic, analgesic, antitussive, expectorant, anticonvulsant, antiepileptic, antihypertensive, anti-inflammatory, or antiviral.
